# EUROPEAN PATENT APPLICATION

(11) **EP 0 709 454 A2**
(43) Date of publication of application: **01.05.1996**
(21) Application number: 95307521.5
(22) Date of filing: 24.10.1995
(51) Int. Cl.: C12M 1/34, C12M 1/22, C12M 1/24, G01N 21/00

(54) **Culture observation vessel and observation device**

(30) Priority: 25.10.1994 JP 260309/94
(71) Applicant: HAMAMATSU PHOTONICS K.K., Shizuoka-ken (JP)
(72) Inventor: Masuko, Masayuki, c/o Hamamatsu Photonics K.K., Hamamatsu-shi, Shizuoka-ken (JP); Suzuki, Makoto, c/o Hamamatsu Photonics K.K., Hamamatsu-shi, Shizuoka-ken (JP); Hayakawa, Tsuyoshi, c/o Hamamatsu Photonics K.K., Hamamatsu-shi, Shizuoka-ken (JP)
(74) Representative: Rackham, Stephen Neil

(57) **Abstract**

A culture vessel (30) having a base section (31) made from a plastic material of a columnar or frusto-conical shape. A side wall (32) is attached to the base section (31) of the vessel to form a basin over the upper surface of the base section (31). The base section (31) acts as an optical wave guide capable of guiding light between the upper and lower surfaces of the base section (31). The lower surface of the base section (31) may be optically connected to the light-entrance window (116) of an image pickup device (11).

## Description

The present invention relates to a culture observation vessel and to an observation device used for observation of cells and microorganisms which emit light.

In medicine, food science, environmental science, and their related fields to which biology is basic, materials under investigation, such as cells and microorganisms, are often observed, still maintained in a culture vessel such as a petri dish and a flask; these materials are cultured in the vessel and then observed directly or after being treated with a suitable process.

One example of observation vessels which a sample can be attached to and cultured in is a one-centimetre height and five-centimetre diameter plastic petri dish of which bottom face is flat and transparent. A sample in this kind of petri dish is visually observed from either above or below, depending on the type of microscope. Microscopes wherein samples are illuminated from above are termed inverted microscopes. A sample is often stained with an appropriate fluorescent dye before observations using either a normal or inverted fluorescent microscope.

When the samples that emit extremely weak fluorescence are observed, additional equipment should be used with the microscope for identifying or quantifying the light emitted from the sample. Chemiluminescence and bio-luminescence may also emit weak light.

Japanese Patent Application Kokai No. HEI-2-500613 describes an observation device which is applied to identify or quantify the light emitted from the sample. This observation device consists of sample holder and the combination of an image intensifier and a CCD camera. The sample holder has an optical fibre glass as its lower section. The image intensifier has a fibre optic face plate as a light entrance window. The sample holder is mounted onto the image intensifier so that the lower face of the sample holder is in direct contact with fibre optic face plate of the image intensifier.

The glass material, described in Japanese Patent Application Kokai No. HEI-2-500613 as the base of the sample holder, is not easily moulded into various forms. For this reason, this material is not suitable for forming desired shapes or for application of mass production techniques. On the other hand, to prevent the environment and other samples from being contaminated, the sample should be maintained within a disposable sample holder. Presently, glass sample holders are also unsuitable because they are difficult to produce in great nu-hers at low costs that permit disposability.

According to the present invention, a culture vessel includes a base section and a side wall. The base section has upper and lower surfaces, and is made from a plastic material that forms an optical waveguide capable of guiding light between the upper and the lower surfaces. The side wall is shaped to fit the base section and form a basin therewith.

According to another aspect of the invention, the base section of the culture observation vessel is formed in either a columnar or conical shape.

According to still another aspect of the present invention , the upper surface of the base section is coated with a polymer, such as polyamino acid and glycoprotein, to enhance efficiency of cell attachment.

According to a further aspect of the present invention, an observation device includes the above-described culture vessel and further includes an image pickup device. This image pickup device has a flat light-entrance window optically connected to the lower surface of the base section so that the device picks up optical images that are formed on its the light-entrance window from the culture vessel.

In another aspect of the present invention, the image pickup device further includes an image intensifier that is located between the culture vessel and the image pickup device. The image intensifier converts an optical image formed on its light-entrance window into electrons, multiplies the electrons, reconverts the electrons into an optical image, and then outputs this resulting optical image to the image pickup device.

According to the present invention, the base section of the vessel is an optical waveguide made from plastic. The upper and the lower surface of the vessel base acts as the input and the output end, respectively, of the optical waveguide. Light-emitting samples attached to the upper surface of the vessel base are in close contact with the input end of the optical waveguide. Therefore, the optical image formed by the sample will be efficiently transferred into the input end even if the optical image has an extremely weak intensity. Finally, the optical image will be transmitted from this end to the output end without loss of the light. The optical image transmitted out from the output end of the vessel preserves positional information in the original form.

The vessel of the present invention is made from plastic, and so is very easy to produce. A commercially available plastic image guide, such as an image plate or optical fibres acting as an image guide, can be easily cut and polished to form a desired shape. Also, plastic image guides can be easily prepared to promote cell attachment using conventional methods. Therefore, optical waveguides formed from commercially available plastic image guides facilitate mass production of observation vessels having a desired shape.

As mentioned above, the base section of the vessel can be easily produced in various forms. Therefore, many types of vessels widely used for tissue cultivation, such as petri dishes and flasks, can be easily produced by attaching a side wall to the base section of the vessel. In addition to serving as a sample holder for observation purposes, the vessel of the present invention can hold tissue cultures on which various biological processes are performed. Accordingly, all operations for preparing a tissue culture for observations can be performed in a single vessel. That is, a sample can be placed in the observation vessel and then cultured. After the sample is cultivated, the same observation vessel can be mounted to the observation device for observing the sample.

The base section of the culture observation vessel can be formed in a plate shape, whether circular or polygonal. Also, the base section can be formed in a column shape or a tapered shape. In a column shape, its upper and lower surfaces will have the same diameter. It should be noted that the upper and the lower surfaces serve as the input and the output surfaces, respectively, of the optical waveguide.

Forming the base section in a tapered shape may be advantageous under certain circumstances. For example, assume that the observation device has a light source with its surface area smaller than the surface area of the sample. When the upper surface is in close contact with the sample and the lower surface is in contact with the light source of the observation device, the base section (i.e., the optical waveguide) must be formed with its upper surface larger than its lower surface. In this way, the optical image from the sample can be reduced and transmitted out of the vessel. In contrast, the optical image of the sample may be enlarged by forming the optical waveguide with its upper surface smaller than its lower surface.

The base section of the culture observation vessel can be formed from any well-known plastic optical waveguide. The fibre core and the other portions that transmit light can be made from any well-known transparent polymer, such as the homopolymer of methyl methacrylate (polymethyl methacrylate), the copolymer of methyl methacrylate and acrylic acid having another functional group, and polycarbonate produced from the ester exchange reaction between phenol ester and 2,2-bis-(4-hydrozyphenol) propane.

Also, the surface of the plastic image guide may be coated with a polyamino acid and a glycoprotein to tightly attach cells to the surface. Commercially available plastic products for tissue culture are often treated with these materials. This means that the materials are easily obtainable and the coating processes are easy to perform.

Any well-known glycoprotein and polyamino acid can be available for coating the surface of the plastic image guide, including the fibre core and the other portions that transmit light, as long as these compounds are harmless to the sample cells and optically inactive. Poly-L-lysine, collagen, and gelatin are examples for polyamino -acid type coating substances. Fibronectin, for example, is used for glycoprotein type coating substances.

In fact, Animal Cell Culture. A Practical Approach (R. I. Freshney ed., IRI Press, Oxford, UK, 1989) describes that plastics, such as polystyrene, polyethylene, polycarbonate, polymethyl methacrylate (Perspex), polyvinyl chloride (PCV), Teflon, cellophane, and cellulose acetate, are suitable for the attachment of animal cells if used correctly. This reference also discusses the surface treatment conditions necessary for the attachment of animal cells. Because the surfaces of both animal cells and the above-listed plastics are negatively charged, electrostatic repulsion may occur, interfering with attachment of cells to the plastic surface. To overcome this interference the surface of the above-listed plastics can be coated with collagen and plyamino acids to make them suitable for cell attachment. A tissue culture grade collagen is commercially available and easy to obtain.

On the other hand, Methods in Enzymology, Vol. 58, Cell Culture (Jakoby, W. B. & Pastan, I. H. ed., Academic Press, New York, USA, 1979) describes that it is advantageous to use polystyrene vessels for conferring disposability to cell culture. Also, this reference notes that the surface of the vessel is harmless to cells and optically inert, the vessel can be used for the attachment of cells.

The optical connection between the base section of the vessel and the light-entrance window of the image pickup device insures that a weak optical image emitted from the sample on the upper surface of the base section will efficiently be transmitted to the light-entrance window of the image pickup device without loss of the positional information in the optical image. The pickup device may be equipped with an image intensifier in front of the device. This image intensifier converts the photon flux of the optical image into electrons, multiplies the electrons, and then reconverts the electrons into output light signals of the optical image.

It is also desirable that the pickup device can be provided with an internal photoelectron effect-type detector, including CCD camera, cooled CCD camera, various vidicons, and other TV cameras. High-sensitive observations can be performed using a CCD camera as a detector. The image pickup device may consist of an image intensifier and a TV camera, wherein the light-entrance window of the image intensifier is optically connected to the base section of the observation vessel. One of the internal photoelectron effect-type detectors listed above is a candidate for the TV camera. With this configuration, the TV camera can pick up the optical image, the light intensity of which is enhanced by the image intensifier. This enables observation of samples that emit extremely weak light, without loss of the positional information of the original image.

The advantages and characteristics of the present invention will be more apparent from reading the following description of the preferred embodiment with drawings in which:
Fig. 1 is a side view of the observation device according to an embodiment of the present invention with an area exposed near the light-entrance window of an image intensifier;
Fig. 2 is a cross-sectional view showing details of the components near the light-entrance window;
Figs. 3 (a) and (b) are a plan view and a cross-sectional view, respectively, showing details of a petri dish used for an embodiment of the present invention;
Figs. 4 (a) and 4 (b) are a plan view and a cross-sectional view, respectively, showing how the actual samples are accommodated in a petri dish; and
Fig. 5 is a perspective view showing a flask as an observation vessel according to the present invention.

The device was used to observe samples of coliform bacterium containing a pUCD plasmid with a lux operon that produce a catalyst for luminescent reaction. Isopropy-1-thio-b-D-galactopyranoside (IPTG) serves as an inducer of the expression of the lux operon, resulting in the light emission from the cells. That is, the observation device of the present embodiment was used to observe this bio-luminescence of the cells.

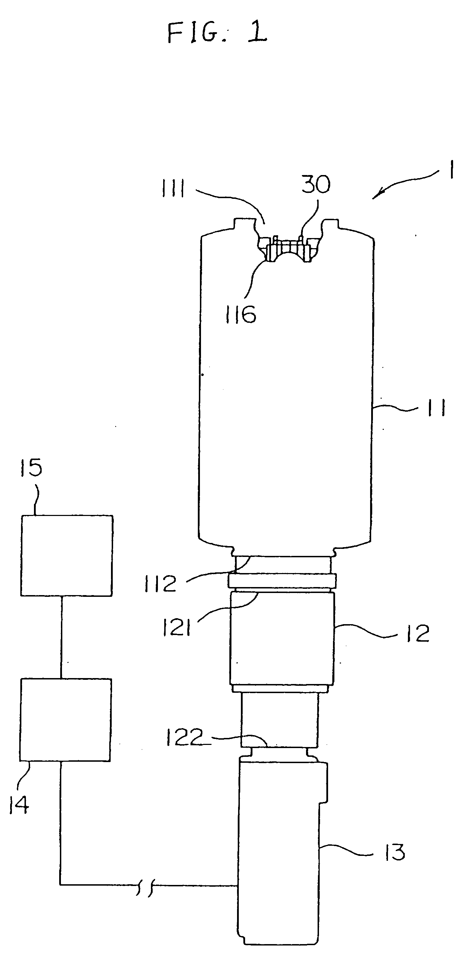

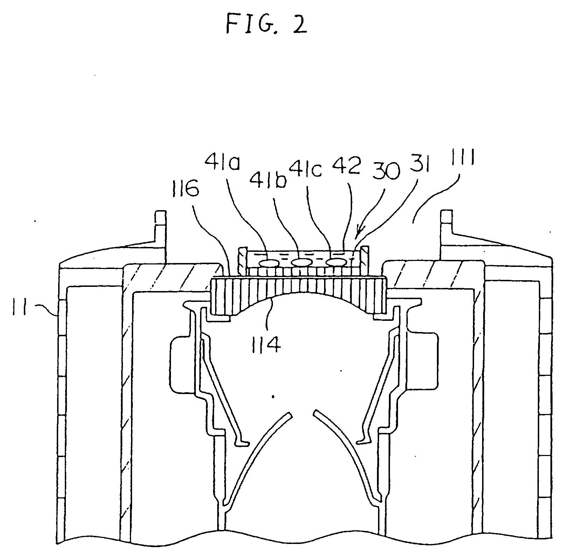

Fig. 1 is a side view of the observation device according to the present embodiment with an area exposed near the light-entrance window 111 of an image intensifier 11. Fig. 2 is a cross-sectional view showing details of components near the light-entrance window 111. As shown in Figs. 1 and 2, the observation device 1 of the present embodiment includes a petri dish 30, a CCD camera 13, a processor 14, and a TV monitor 15. The petri dish 30, image intensifier 11, relay lens 12, and CCD camera 13 are provided in an upright posture.

The petri dish 30 is a culture observation vessel used for containing a culture medium and samples 41 (that is, 41a, 41b, and 41c in the drawing) under investigation. The petri dish 30 has a base section 31 made from a plastic optical fibre plate.

The image intensifier 11 is a cascaded image intensifier system originally developed for particle track imaging. This has an external light-entrance window 116. The light-entrance window 116 is constructed from a fibre glass plate and has a photoelectric face 114 at its lower surface. The petri dish 30 is mounted onto the opening 111 with its base section 31 in intimate contact with the light-entrance window 116. Optical images formed from the samples 41 in the petri dish 30 arrive on the light-entrance window 116 through the base section 31. The image intensifier 11 converts the optical images into electrons, amplifies the electrons, and then reconverts the electrons into an optical image, which is output from the output end 112 of the image intensifier 11.

The relay lens 12 is attached to the output end 112 of the image intensifier 11 through the lens connector 121. Optical images output from the output end 112 are transmitted through the relay lens connector 121 and focused on the light-entrance window of the CCD camera.

The CCD camera is connected with the output end 122 of the relay lens 12. The CCD camera 13 picks up the optical image focused on its light-entrance window, converts the light signal into an electric signal, and then outputs the electric signal to the processor 14. This processor manipulates the signal for data processing. The TV monitor 15 converts the electric signal from the processor 14 into an image.

In short, the observation device of Fig. 1 consists of three portions: (1) the image intensifier 11 intensifying the optical image attained onto its light-entrance window, (2) the relay lens 12 focusing the optical image output from the output end of the image intensifier 11 onto the light-entrance window of the CCD camera 13, and (3) the CCD camera 13 converting the optical image into the electric signal. The signal output from the CCD camera is processed in the processor 14 and transmitted to the TV monitor 15.

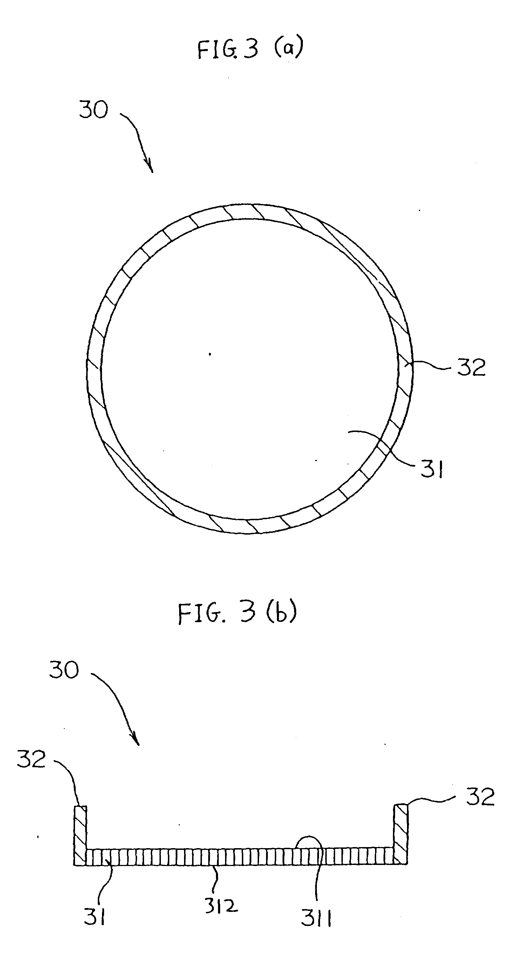

Figs. 3 (a) and 3 (b) are a plan view and a cross-sectional view, respectively, showing details of the petri dish 30 used in the present embodiment. As shown in Figs. 3 (a) and (b), the petri dish 30 includes a base section 31 and a side wall 32. The base section 31 is made from a plastic optical fibre plate formed in a disk shape with thickness of 2.0 mm and diameter of 25.4 mm (1 inch). The side wall 32 is made from plastic or glass formed in a ring shape with inner diameter of 25.4 mm (1 inch) and with thickness of 1.0 mm. Although not shown in the drawings, a lid is provided for covering the petri dish 30 when culturing the samples.

The petri dish 30 is produced in the following manner. First, a plastic optical fibre plate (step index type), of which individual single fibres were 25.0 mm in diameter, is cut to a thickness of 2.0 mm and a diameter of 28.0 mm. This is polished to a diameter of 25.4 mm (1 inch) and a thickness of 2.0 mm to form the disk-shaped base section 31. The plastic optical fibres making up the base section 31 have a core material of polymethyl methacrylate and a clad material of fluorocarbon resin. Light with intensity of 190,000 lux could be transmitted through these optical fibres to emit light with intensity of 12,000 lux.

The side wall 32 is formed from polymethyl methacrylate into a ring shape with a diameter of 25.4 mm (1 inch) and a thickness of 1.0 mm. The side wall 32 is then mounted to the base section 31 to form a basin over the upper surface of the base section 31. The edge is formed by joining the side wall 32 with the base section 31 and adhered tightly with a commercially available adhesive to prevent culture solution from leaking. The petri dish 30 so constructed has a cylindrical shape with an open top, as shown in Fig. 3 (a) and (b).

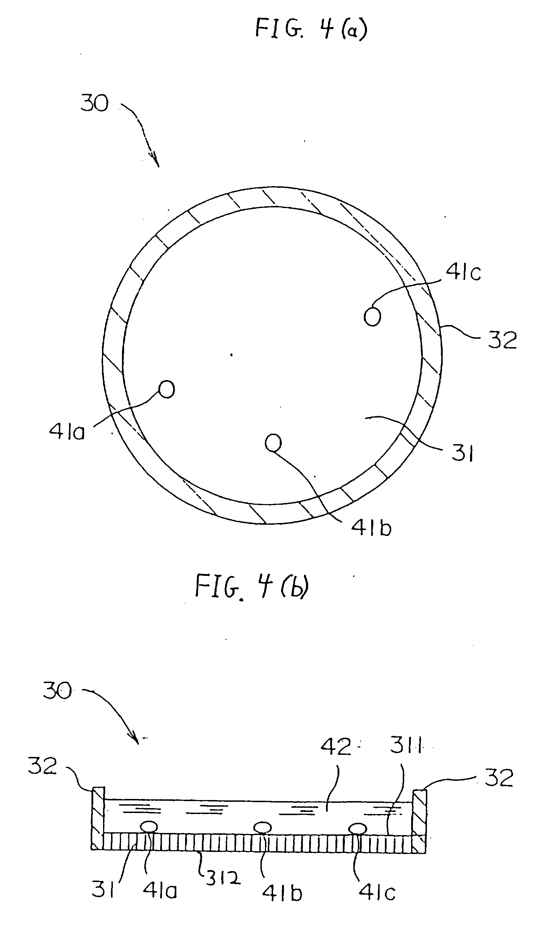

One of the examples that the gene expression of a coliform bacterium was actually observed using the above described observation device will be presented while referring to Figs. 1, 2, and 4. Figs. 4 (a) and 4 (b) are a plan view and a cross-sectional view, respectively, showing how the sample 41 is contained in the petri dish 31.

The method for preparing the sample 41 used in this observation will be explained here. First, the upper surface 311 of the base section 31 was coated with poly-L-lysine. Next, the cells of a coliform bacterium (Escherichia coli JM101) suspended in a culture medium were spread over the inner surface 31 of the vessel 30 so that a part of the cells were attached to the surface 31. Finally, the vessel 30 was washed once with the same fresh medium used above and was filled with about 1 ml of the fresh medium 42 again. The Escherichia coli used in this experiment contains the pUCD plasmid carrying the Vibrio fischeri luxCDABE operon under lacOP control. In Fig. 2, the cells of E. coli is schematically exhibited as samples 41a to 41c. Although, in this experiment, the poly-L-lysine functions as a binding substance for E. coli to attach the surface 31 of the vessel 30,another polyamino acid, such as collagen, gelatin, various glycoproteins, can also be applied for this function.

As shown in Fig. 2, the petri dish 30 containing the sample 41 was mounted onto the light-entrance window 116. The lower surface of the base section 31 was pre-coated with silicon grease to ensure tight sealing between the lower surface of the base section 31 and the outer surface of the light entrance-window 116.

Next, isopropyl-1-thio-b-D-galactopyranoside (IPTG) (not shown in the drawings), which is a reagent for inducing the light emission of E. coli, was added to the culture solution 42 contained in the petri dish 30. About twenty minutes after this treatment, the sample cells 41 began to emit weak light. The optical image so formed from the sample cells 41 was collected into the plastic optical fibre plate of the base section 31 of the vessel 30 and then transmitted through it. The transmitted image is then transferred to the light-entrance window 116 of the image intensifier 11, which is optically connected to the plastic optical fibre plate. In other words, the base section 31 from its upper surface to lower surface served as an image guide for the sample image. Therefore, the weak optical image formed from the sample 41 is directly collected into the light-entrance window 116 and then transmitted to the image intensifier 11 without need for a coupling lens system between the sample and the image intensifier 11 for focusing.

About 100 luminous spots were detected about 30 min after addition of the IPTG. This means that each point should correspond to each cell of E. coli. During this observation time, no appreciable growth of the cells can occur. The temporal monitoring of each luminous spot allows understanding of the expression pattern of the lux gene.

The present invention can be used for other applications other than the observation of gene expression. For example, immunoassays can be performed using the vessel and observation device according to the present invention. As an exemplary method of immunoassays, an enzyme-linked immunosorbent assay (ELISA) using particles as the solid phase is known. At the final step of the assay, the activity of the enzyme adsorbed on the particles is determined, indicating how much of the target molecule exists in an analytical sample. At this step, the present invention can be applied if the enzyme catalyzes a luminescent reaction (e.g. horseradish peroxidase); the particles are mounted on the petri dish of the present invention and this vessel is placed on the light-entrance window of the image intensifier. After adding a reagent for inducing light emission, the image of the particles can be obtained and analyzed.

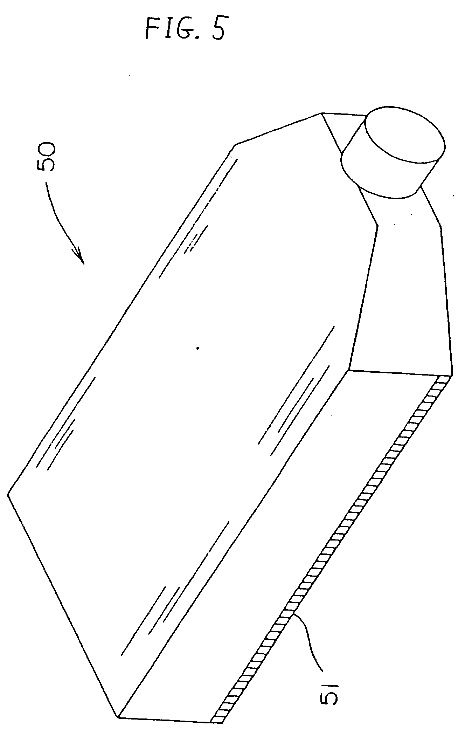

Also, the present embodiment can be applied to vessels other than the petri dish 30. One of the examples is shown in Fig. 5, a perspective view of flask 50. This flask 50 has a base section 51 made from a disk-shaped plastic optical fibre plate of which thickness is about 2 mm. The sample is attached to the inner surface of the base section 51. A culture medium suitable for the sample is contained in the flask. Light emitted from the sample can be observed after the flask 50 is mounted on the light-entrance window of the image intensifier like the petri dish 30 in Figs 1 and 2. Similarly, frusto-conical shaped plastic optical fibre plate can be used as the base sections of petri dish and flask.

As previously described in detail, the present invention provides a culture observation vessel wherein the base section, which serves as an optical waveguide, is made from plastic. Thus the vessel can be easily made in any desired shape. Ordinarily used plastic has a low melting point, 200uBC or less, and so can be easily moulded into a desired shape. Also, plastic materials can be joined together with each other by either melting or using adhesives. In comparison, glass materials are manufactured through troublesome processes. Thus, skilful craftsmen are essential for this work. Furthermore, glass materials generally tend to be more easily broken by shocking than plastic materials. This is a more important point to manipulate dangerous, toxic and pathogenic organisms included in the vessels. In summary, plastic is superior to glass materials for culture vessels.

Because the observation vessel of the present invention is made from plastic, it can be produced inexpensively. Therefore, if previously sterilized and coated with a binding substance such as collagen, the plastic observation vessels may be used only once and then discarded.

A great variety of plastic materials are now available. Thus the observation vessels of the present invention will be able to be manufactured with these materials, depending upon the specifications of the vessels.

According to the present invention, the lower face of the base section in the vessel is optically connected to an image pickup device, indicating that the images made from samples can be easily and efficiently observed without loss of positional information included in the images. Especially, this characteristic should exert its effectiveness when observed samples only emit light, such as bio-luminescence and chemiluminescence, that has an extremely low intensity.

## Claims

1. A culture vessel (30) for containing cells and microorganisms, the culture vessel (30) comprising:
a base section (31) having an upper surface and a lower surface, the base section being made from a plastic material that forms an optical waveguide capable of guiding light between the upper and the lower surfaces; and
a side wall (32) attached to said base section (31) to form a basin over the upper surface of said base section (31).

2. A culture observation vessel (30) according to claim 1, wherein the base section (31) has a columnar shape.

3. A culture observation vessel (30) according to claim 1, wherein the upper surface of said base section has a frusto-conical shape.

4. A culture observation vessel (30) according to any one of the preceding claims, wherein the upper surface of the base section (31) is coated with at least one of a polyamino acid and a glycoprotein.

5. A culture observation vessel according to any one of the preceding claims, wherein said side wall (32) is in intimate contact with said base section (31) along a perimeter of said base section (31).

6. An observation device (1) for quantitative and qualitative analysis of light-emitting samples by observing light emitted from the samples, the observation device including a culture vessel (30) according to any one of the preceding claims, and
an image pickup device (11) having a flat light entrance window (116) optically connected to the lower surface of the base section (31) of said vessel (30), the image pickup device (11) picking up optical images that are transferred from the culture vessel (30) to the light entrance window (116) of the device.

7. An observation device according to claim 6, wherein the image pickup device (11) further includes an image intensifier to which is disposed a light-entrance window (116), the image pickup device converting an optical image entering into the light-entrance window (116), converting the image into electrons, multiplying the electrons, reconverting multiplied electrons into an optical image, and outputting the optical image to a TV camera (13).
